# EUROPEAN PATENT SPECIFICATION

(11) **EP 1 110 518 B1**
(45) Date of publication and mention of the grant of the patent: **10.11.2004**
(21) Application number: 00204662.1
(22) Date of filing: 20.12.2000
(51) Int. Cl.: A61F 5/56

(54) **Device for preventing bruxism**
Vorrichtung zur Verhinderung des Zähneknirschens
Dispositif anti-bruxisme

(43) Date of publication of application: 27.06.2001
(73) Proprietor: Burger, Michael, Albertus, NL-5622 AW Eindhoven (NL)
(72) Inventor: Burger, Michael, Albertus, NL-5622 AW Eindhoven (NL)

(56) References cited:
- WO-A-94/28828
- US-A- 5 078 153
- US-A- 5 265 624

## Description

The invention relates to a device for preventing bruxism, having a splint that can be fixed to the teeth with help of mounting elements of electrical conductive material, said mounting elements being electrically connected to a control unit which delivers electrical pulses when bruxism occurs. This device is known from EP 0.654.981 B1.

In order to prevent bruxism, a splint is used. For example the splint is a synthetic resin shaped plate most frequently worn on the roof of the mouth and fixed to the teeth of the upper jaw. Bruxism, also known as teeth grinding or teeth clenching, is any non-physiological activity of the teeth and for example occurs when someone is asleep. It may cause a disturbed nightrest and it may even damage teeth and molars.

In the known device, as soon as bruxism starts, the patient receives electrical pulses with help of the electrically conductive mounting elements. These mounting elements are the anchors of the splint. Bruxism subsequently stops and the normal physiology is restored.
For detecting bruxism the known device is provided with pressure elements. These elements are mounted to the splint. Said pressure elements are connected to an electric control unit and a pulse generator to send pulses to the mounting elements.

Due to the presence of said pressure elements, it appeared that the known device is rather complicated and difficult to manufacture. The use of pressure elements further has the disadvantage that these elements are not able to detect grinding as well as clenching. As a consequence two kinds of pressure elements in the splint are necessary.

The pressure difference between rest and stress of a patient must be detected very accurately in order to obtain an optimal efficiency of the device. This may give rise to imperfections, in particular in a mass manufacturing process. This is disadvantageous.

It is an object of the invention to mitigate these disadvantages.

According to the invention, the device mentioned in the opening paragraph is characterised in that the splint comprises at least one receiving antenna that is sensible for receiving electrical signals from jaw muscles, which antenna is located on the splint which in operation is present in the mouth and which antenna is electrically connected to the control unit, which antenna is located near the edge of the splint near the mounting elements.

In the device according to the invention, during bruxism or teeth clenching, the electrical activity of the jaw muscles is detected with help of the antenna. Due to the fact that the antenna is located near the edges of the splint in the neighbourhood of the mounting elements, electrical signals that are transmitted as a result of the changing activity of the jaw muscles during bruxism can be detected easily.
Namely, the antenna, the mounting elements and the jaw muscles are located close to each other near the edge of the splint.

A device according to the invention comprises only a few elements, since the use of special pressure elements and its corresponding connection wires is avoided.
As a result thereof, the device can be manufactured easily. Construction and measuring failures are reduced to a minimum. Also, the device according to the invention is very hygienic.

The electrical signals that are transmitted by the jaw muscles during bruxism are received by the antenna and subsequently transmitted further to the control unit. The control unit measures the signal. As soon as said signal exceeds a predetermined threshold value, the control unit sends an electrical stimulus pulse to the mounting elements. The patient then receives a small electrical shock, as a result of which the bruxism stops.

Then the neuromuscular equilibrium in the muscle system of jaw, head and cervix is restored quickly in comparison with the known device.
It appeared that with help of the device according to the invention other activities of the human body react in a positive way when the unbalance of the neuromuscular operation of head and cervix comes to an end.

The human body again finds its balance between activity and rest. Various pathological physical actions (such as a high blood pressure) and mental functions (such as sleep disorder) are eliminated.

The activity of the jaw muscles can be measured with help of the antenna with great accuracy. The antenna itself for example consists of a thin metal layer, which is integrated in the wall of the synthetic resin of the splint. In a preferred embodiment the receiving antenna is integrated in the mounting elements made of an orthodontic metal.

The advantage of this embodiment is that the device can be manufactured in a simple way. Namely, to the splint no additional provisions are necessary.

An additional advantage is that the mounting elements are located in a predetermined and fixed position with respect to the jaw muscle system.

The antenna is electrically connected to the control unit. This electrical connection for example is a wire, but this connection can also be wireless, as well known in the art. This also is applicable to the connection between the control unit and the mounting elements.

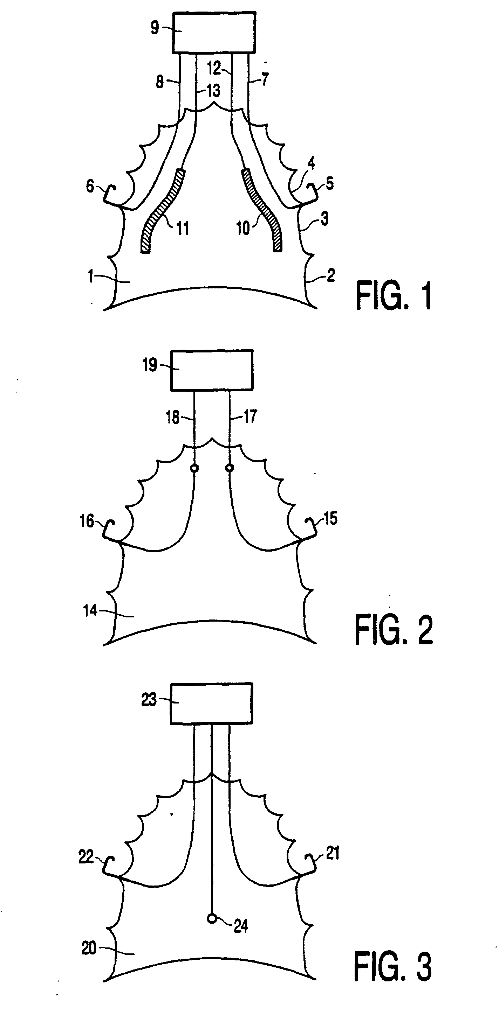

Embodiments of the device according to the invention are shown in the drawing, in which figure 1 shows schematically a first embodiment of the invention.

Figure 2 shows a second embodiment in which the mounting elements are antennas at the same time.

Figure 3 shows the embodiment according to figure 2, in which the device is provided with a third orthodontic metal anchor projecting from the splint.

The device according to figure 1 comprises a splint 1 of synthetic resin material. The splint is a plate that is clamped to the teeth of the upper jaw and pressed to the roof of the mouth. The edge of the splint comprises indentations corresponding to the shape of the teeth and molars. By way of example 2,3 and 4 indicate a few of such indentations. The splint can also be made as a cover over the chewing planes of the molars and over the cutting sections of the teeth.

The splint further comprises two mounting elements 5 and 6, both made of orthodontic metal. With help of said mounting elements the splint is fixed to the teeth. In this embodiment said mounting elements are located near the teeth and molars P2SD, P2SS, M1SD and M1SS.

The splint is fixed properly to the teeth with help of the mounting elements. A patient is able to detach the splint from the teeth in an easy way.
Said mounting elements 5 and 6 of orthodontic metal are embedded or integrated in the synthetic resin material of the splint. The elements 5 and 6 are connected to the electrical control unit 9 via wires 7 and 8 respectively.

During bruxism the electrical control unit emits electrical pulses to the mounting elements 5 and 6. The splint further comprises two receiving antennas 10 and 11, which are able to receive electrical signals from the jaw muscles. Said antennas are connected to the electric control unit 9 via wires 12 and 13. Said antennas of electrically conducting material are located near the mounting elements and near the edge of the splint.

In this embodiment the antennas are longitudinally shaped and enclosed by the synthetic resin material of the splint.

The electrical signals originate from the jaw muscles. These are located at a short distance from the mounting elements. During bruxism or teeth clenching a change in the electrical activity of the jaw muscles occurs. The antennas immediately detect the change in signal level. This signal then is sent to the control unit, which subsequently transmits electric pulses to the mounting elements 5 and 6. This is a small electric shock for the patient and bruxism stops immediately.

In the embodiment according to figure 2 the splint is indicated by reference numeral 14. The end parts of the wire shaped mounting elements 15 and 16 are fully embedded in the hardened synthetic resin of the splint. These elements are receiving antennas at the same time. These elements are connected to the electrical control unit 19 by wires 17 and 18.

The antennas receive the signals that are transmitted by the jaw muscles. The currents have an intensity of a few nano amperes in a frequency range of 300 to 700 Hz. If said signal continues to be present for more than about three seconds, the control unit transmits current pulses to elements 15 and 16 having an intensity sufficient to stop bruxism.

In the embodiment according to figure 3, 20 indicate the splint and 21 and 22 indicates the mounting elements.

These elements are connected to control unit 23.

The splint 20 is provided with an orthodotic metal anchor 24 projecting from the splint and resting against the roof of the mouth. Said anchor (ground electrode) serves as an electric zero value with respect to the elements 21 and 22. Low frequent noise signals (50Hz) then are eliminated easily.

## Claims

1. Device for preventing bruxism, having a splint (1) with help of mounting elements (5,6) of electrical conductive material, said splint (1) being fixable to teeth with the help of said mounting elements (5,6), said mounting elements (5,6) being electrically connected to a control unit (9), which delivers electrical pulses when bruxism occurs, **characterised in that** the splint (1) comprises at least one receiving antenna (10,11) that is sensible for receiving electrical signals emitted by jaw muscles, which antenna (10,11) is located on the splint which in operation is present in the mouth and which antenna is electrically connected to the control unit (9), which antenna (10,11) is located near the edge of the splint (1) near the mounting elements.

2. Device as claimed in Claim 1, **characterised in that** the mounting elements (5,6) are performed as antennas.

3. Device as claimed in claim 1 or 2, **characterised in that** the splint (1) is provided with an anchor (24), projecting from said splint (1) and resting against the roof of the mouth, which anchor (24) is electrically connectable to the control unit (9).

## Patentansprüche

1. Vorrichtung zur Verhinderung des Zahnenknirschens mit einem Träger (1), der mit Befestigungselementen (5,6) aus elektrischem leitendem Material versehen ist, welcher Träger (1) den Zahnen mit Hilfe der Befestigungselementen verbunden werden kann, wobei die Befestigungselementen (5,6) mit einer elektrischen Kontrolleinheit (9) verbunden sind, welche Kontrolleinheit elektrische Pulse zu den Befestigungselementen abgibt beim anfangen des Zahnenknirschens, **dadurch gekennzeichnet, dass** der Träger (1) mindestens eine Empfangantenne (10, 11) enthält, die für den Empfang der von den Kaumuskeln gesendeten elektrischen Signalen empfindlich ist, welche Antenne (10, 11) sich auf dem Träger (1) befindet, welcher Träger (1) sich während des Betriebs der Vorrichtung im Mund befindet und mit der Kontrolleinheit (9) elektrisch verbunden ist, welche Antenne (10,11) am Rande des Trägers (1) in der Nähe der Befestigungselementen angeordnet ist.

2. Vorrichtung gemäss Anspruch 1, **dadurch gekennzeichnet, dass** die Befestigungselementen (5,6) als Antenne ausgeführt sind.

3. Vorrichtung gemäss Anspruch 1 oder 2, **dadurch gekennzeichnet, dass** ein Anker (24) auf demTräger (1) angeordnet ist, welches Anker (24) im Betrieb gegen den Gaumen presst und elektrisch mit der Kontrolleinheit (9) verbunden ist.

## Revendications

1. Dispositif anti-bruxisme comprenant une attelle (1) pourvue d'armatures (5,6) métalliques pour conduire des courants électriques, l'attelle (1) est fixable aux dents à l'aide des armatures (5,6) qui sont connectées électriquement avec un élément de control (9) pour conduire des coups électriques pendant de grincements de dents, **caractérisé en ce que** l'attelle (1) comprend du moins une antenne (10,11) pour recevoir des signeaux électriques émis par des masséters, l'antenne (10,11) est située à l'attelle (1) qui se trouve dans la bouche et est connectée avec l'élément de control (9), l'antenne (10,11) est présent au bord de l'attelle (1) aux environs des armatures.

2. Dispositif selon la revindication 1, **caractérisé en ce que** les armatures (5,6) ont la fonction d' une antenne.

3. Dispositif selon la revindication 1 ou 2, **caractérisé en ce que** l'attelle (1) comprend un grappin (24) projeté au palais de la bouche, le grappin (24) peut être connecter à l'élément de control (9).
